# EUROPÄISCHE PATENTSCHRIFT

(11) **EP 3 964 118 B1**
(45) Veröffentlichungstag und Bekanntmachung des Hinweises auf die Patenterteilung: **26.02.2025**
(21) Anmeldenummer: 21186407.9
(22) Anmeldetag: 19.07.2021
(51) Int. Cl.: A61B 1/06, A61B 1/00, A61B 1/07, F21V 8/00, G02B 6/42

(54) **BELEUCHTUNGSVORRICHTUNG MIT LICHTLEITERERKENNUNG**
ILLUMINATION DEVICE HAVING LIGHT CONDUCTOR DETECTION
DISPOSITIF D'ÉCLAIRAGE À DÉTECTION DE GUIDE D'ONDES OPTIQUES

(30) Priorität: 03.09.2020 DE 102020123031
(43) Veröffentlichungstag der Anmeldung: 09.03.2022
(73) Patentinhaber: Karl Storz SE & Co. KG, 78532 Tuttlingen (DE)
(72) Erfinder: HINDING, Thomas, 78532 Tuttlingen (DE); GLÖGGLER, Bernhard, 78532 Tuttlingen (DE); GÖBEL, Werner, 78532 Tuttlingen (DE); PFAU, Teresa, 78532 Tuttlingen (DE)
(74) Vertreter: Patent- und Rechtsanwälte Behrmann Wagner PartG mbB

(56) Entgegenhaltungen:
- DE-A1- 102009 005 839
- US-A1- 2011 257 483
- US-A1- 2013 345 517

## Beschreibung

Die vorliegende Erfindung betrifft eine Beleuchtungsvorrichtung für ein Endoskop mit integrierten Detektionsmitteln zur Lichtleitererkennung, sowie ein Verfahren zum Betreiben der Beleuchtungsvorrichtung.

In der modernen Endoskopie ist die Bereitstellung einer Lichtquelle zur Ausleuchtung bei der Untersuchung von im Allgemeinen schwer zugänglichen Hohlräumen unerlässlich. Neben der Bereitstellung einer Lichtquelle an einem distalen Ende eines Endoskops zur Einführung in den zu untersuchenden Hohlraum ist es bereits hinreichend bekannt, eine externe Lichtquelle bzw. eine diese aufweisende Beleuchtungsvorrichtung vorzusehen und Licht über ein proximales Ende des Endoskops mittels eines Lichtleiters aufweisend darin angeordnete lichtleitende Fasern zum distalen Ende bzw. zur Endoskopspitze zu übertragen, wo dieses zur Hohlraumbeleuchtung austritt. Dies birgt neben einer kleineren Bauform des Endoskops den Vorteil der Vermeidung eines größeren Wärmeeintrags in den zu untersuchenden Hohlraum.

Neben Halogen-Glühlampen oder Lichtbogenlampen ist es ebenfalls bereits bekannt, Leuchtdioden (LEDs) als Lichtquellen in der Beleuchtungsvorrichtung vorzusehen. Das durch die Beleuchtungsvorrichtung bereitgestellte Licht wird üblicherweise an einem vorzusehenden Lichtleiteranschluss, beispielsweise einer Steckkupplung, in den mit dem Endoskop verbundenen oder damit verbindbaren Lichtleiter eingekoppelt.

Die bisher bekannten Lichtquellen bzw. diese aufweisenden Beleuchtungsvorrichtungen sind üblicherweise auf den jeweils zu kontaktierenden Lichtleiter abgestimmt. Insbesondere ist eine Emissionsfläche der Lichtquelle auf die Transmissionsfläche des damit zu kontaktierenden Lichtleiters abgestimmt. Hierdurch wird eine einfache und insbesondere energieeffiziente Ausgestaltung der Beleuchtungsvorrichtung ermöglicht. Gleichzeitig führt dies jedoch zu einer Beschränkung der Einsatzmöglichkeiten. Dies insbesondere da in der Regel je nach Einsatzzweck und bedingt durch anatomische Gegebenheiten verschiedene Lichtleiter und Endoskope verwendet werden. Beispielsweise werden in der Laparoskopie vorzugsweise Lichtleiter mit 4,8mm Durchmesser eingesetzt, während bei der Otoskopie deutlich dünnere Lichtleiter eingesetzt werden.

Die DE 10 2013 113 511 A1 offenbart ein Endoskop mit einem Lichtfaserbündel zur Übertragung von Licht von einem proximalen Ende zu einem distalen Ende, wobei das proximale Ende ein zusammenhängendes Lichtfaserbündel und das distale Ende mehrere arbiträr verteilte Teilbündel oder auch Einzelfasern aufweist. Die Einkopplung von Licht erfolgt mittels einer dem proximalen Ende zugeordneten arrayartigen LED Lichtquelle mit Einzellichtquellen, wobei durch die arbiträre Verteilung der Teilbündel und der hierdurch erzielbaren Verteilung der aktivierten Einzellichtquellen eine Optimierung der Wärmeverteilung in der Lichtquelle angestrebt wird. Eine Ansteuerung der Einzellichtquellen erfolgt durch eine Steuereinheit unter Verwendung einer Zuordnungsfunktion, welche die Zuordnung zwischen einzelnen Teilbündeln zu den proximalen Endflächen der Fasern bestimmt, die den jeweiligen Einzellichtquellen zugeordnet sind. Die Zuordnungsfunktion wird mit einer separaten Prüfvorrichtung bei der Montage des Endoskops bestimmt, wobei die Prüfvorrichtung externe Fotodioden zur Erfassung eines jeweiligen Lichtaustritts am distalen Ende der Teilbündel des Endoskops aufweist.

Die US 2011/0257483 A1 offenbart eine Vorrichtung aufweisend eine LED Matrixlichtquelle, eine dieser zugeordneten Fokussiereinheit und einen bezüglich einer Emissionsfläche der Fokussiereinheit anordenbaren Lichtleiter eines Endoskops. Eine der LED Lichtquelle zugeordnete Steuereinheit ist derart ausgebildet, dass diese ein Auslesen eines Speichers in einem jeweiligen Endoskop vornimmt und basierend darauf eine Ansteuerung der Lichtquelle erfolgt.

Die US 2005/0140270 A1 offenbart eine Beleuchtungseinheit aufweisend ein LED Matrixfeld mit einer Vielzahl von LED Lichtquellen und einen diesen zugeordneten Lichtleiter, wobei der Lichtleiter durch eine vorgesehene Stützstruktur zwischen seinem ersten und zweiten Ende stabilisiert wird, und wobei die Vorrichtung eine Wärmesenke aufweist, die thermisch an eine Leiterschicht der LED Lichtquellen angekoppelt ist.

Ausgehend von dem bekannten Stand der Technik liegt der vorliegenden Erfindung die Aufgabe zugrunde, eine Beleuchtungsvorrichtung und ein Verfahren zum Betreiben einer Beleuchtungsvorrichtung für ein Endoskop bereitzustellen, welches die obigen Nachteile des Standes der Technik adressiert. Insbesondere soll eine verbesserte Beleuchtungsvorrichtung und ein Verfahren zum Betreiben einer Beleuchtungsvorrichtung bereitgestellt werden, welches eine erweiterte Einsetzbarkeit und gleichzeitig eine energieeffiziente und einfache Ausgestaltung ermöglicht.

Gelöst wird diese Aufgabe durch die Vorrichtung und das Verfahren gemäß den unabhängigen Ansprüchen. Die abhängigen Ansprüche beschreiben vorteilhafte Weiterbildungen der vorliegenden Erfindung. Die Erfindung adressiert zudem weitere Probleme, wie aus der nachfolgenden Beschreibung hervorgeht.

In einem ersten Aspekt betrifft die Erfindung eine Beleuchtungsvorrichtung für ein Endoskop aufweisend Beleuchtungsmittel zur Einkopplung von Licht in einen mit der Vorrichtung verbindbaren Lichtleiter und aufweisend ein LED Matrixfeld umfassend eine, eine Emissionsfläche bildende, Vielzahl von LED Pixeln, einen den Beleuchtungsmitteln zugeordneten Lichtleiteranschluss zur Positionierung einer endseitig am Lichtleiter angeordneten lichtleitenden Transmissionsfläche im Wesentlichen orthogonal zu einer Hauptabstrahlrichtung der Emissionsfläche des LED Matrixfelds, wobei die Vorrichtung Detektionsmittel aufweist, welche zur Erfassung von in Hauptabstrahlrichtung auf nicht-lichtleitende Bereiche des Lichtleiters ausgerichteten LED Pixeln, der Emissionsfläche ausgebildet sind, und dass die Vorrichtung eine Steuereinheit aufweist, welche zur selektiven Ansteuerung der LED Pixel des LED Matrixfelds für die Lichteinkopplung in den Lichtleiter ausgebildet ist, derart, dass die in Hauptabstrahlrichtung auf die nicht-leitenden Bereiche des Lichtleiters ausgerichteten LED Pixel wenigstens teilweise mit geringerer Leistung angesteuert oder deaktiviert werden.

Die bereitgestellte Vorrichtung ermöglicht eine Erkennung von nicht-lichtleitenden Bereichen eines an der Beleuchtungsvorrichtung angeordneten Lichtleiters und somit eine Erkennung von unterschiedlichen Lichtleitern hinsichtlich deren Transmissionsfläche bzw. hinsichtlich deren die Transmissionsfläche umgebenden nicht-lichtleitenden Bereiche. Bei der Lichteinkopplung in den Lichtleiter kann somit eine selektive, d.h. individuelle, Ansteuerung der einzelnen LED Pixel zur Lichtemission erfolgen, wobei die auf nicht-lichtleitenden Bereiche des Lichtleiters ausgerichteten LED Pixel wenigstens teilweise mit geringerer Leistung angesteuert oder deaktiviert werden. Hierdurch wird der Lichteintrag in nicht-lichtleitende Bereiche und somit eine unerwünschte thermische Belastung des Lichtleiters und insbesondere eines in der Regel metallischen Steckers des Lichtleiters reduziert bzw. vermieden. Zudem wird ein energieeffizienter Betrieb der Beleuchtungsvorrichtung ermöglicht, da eine ideale Ausnutzung der Lichtquelle für den jeweils verbundenen Lichtleiter erzielbar ist und nicht nur die Bereitstellung von einer zu geringen lichtemittierenden Emissionsfläche für den verbundenen Lichtleiter, sondern insbesondere auch die Bereitstellung von einer zu großen lichtemittierenden Emissionsfläche vermieden wird. Eine ansonsten unter Umständen vorzusehende Kühlung für den Lichtleiter bzw. Lichtleiterstecker kann ebenfalls vermieden werden, wodurch neben den vorgenannten Vorteilen eine einfache und platzsparende Ausgestaltung der Beleuchtungsvorrichtung erzielbar ist.

Unter "LED Matrixfeld" wird im Folgenden ein LED Matrix Array verstanden, welches eine Vielzahl von darauf insbesondere gitterartig angeordneter und individuell ansteuerbarer LED Pixel aufweist. Diese sind vorteilhaft durch einzelne, weißlichtemittierende LED Chips gebildet. Das LED Matrixfeld weist vorzugsweise zwischen 500 und 4500, mehr bevorzugt zwischen 1000 und 4500 LED Pixel auf. Die einzelnen LED Pixel bilden eine zusammenhängende Emissionsfläche des LED Matrixfelds. Die einzelnen LED Pixel sind vorzugsweise wenigstens teilweise hinsichtlich deren Lichtintensität bzw. Lichtemission individuell ansteuerbar ausgebildet. Dies kann mit an sich bekannter Ansteuerung wie beispielsweise einer Pulsweitenmodulation erfolgen.

Unter "Hauptabstrahlrichtung" der Emissionsfläche wird im Folgenden eine Richtung orthogonal zur durch die LED Pixel gebildete Emissionsfläche verstanden, und in welche eine Lichtemission der LED Pixel erfolgt.

Unter "nicht-lichtleitende Bereiche" des Lichtleiters werden im Folgenden insbesondere gegenüber einer lichtleitenden Transmissionsfläche deutlich reduziert lichtleitende und im Wesentlichen nicht lichtleitende Teile des Lichtleiters verstanden. Diese umfassen insbesondere einen endseitig am Lichtleiter angeordneten Lichtleiterstecker bzw. eine Lichtleiterhülse, welche die lichtleitende und zusammenhängende Transmissionsfläche umgibt. Vorzugsweise sind die nicht-lichtleitenden Bereiche insbesondere entgegen einer Hauptabstrahlrichtung der Emissionsfläche reflektierende Bereiche des Lichtleiters. Die Transmissionsfläche des Lichtleiters ist vorzugsweise aus einer Vielzahl lichtleitender Faserenden gebildet, welcher zur Lichteinkopplung im Wesentlichen orthogonal zur Hauptabstrahlrichtung der Emissionsfläche angeordnet werden.

Unter auf einen nicht-lichtleitenden Bereich "ausgerichtet" wird insbesondere verstanden, dass Emissionsstrahlung des jeweiligen LED Pixels in Hauptabstrahlrichtung auf wenigstens einen Teilbereich eines nicht-lichtleitenden Bereichs trifft. Dies bedeutet vorzugweise, dass in Draufsicht auf die Emissionsfläche, entlang der Hauptabstrahlrichtung, wenigstens ein Teilbereich, vorzugsweise wenigstens 30%, mehr bevorzugt wenigstens 50% des jeweiligen LED Pixels durch einen nicht-lichtleitenden Bereich des Lichtleiters verdeckt bzw. abgedeckt ist.

In einer bevorzugten Ausführungsform ist die Steuereinheit ausgebildet, alle in Hauptabstrahlrichtung auf einen nicht-lichtleitenden Bereich des Lichtleiters ausgerichtete LED Pixel bei der Lichteinkopplung in den Lichtleiter zu deaktivieren. Hierdurch kann ein unerwünschter Wärmeeintrag in nicht-lichtleitende Bereiche des Lichtleiters vermieden und die Effizienz bei der Lichteinkopplung weiter optimiert werden.

In einer bevorzugten Ausführungsform ist die Steuereinheit zur Erkennung einer die Transmissionsfläche umgebenden nicht-lichtleitenden Außenkontur des Lichtleiters basierend auf von den Detektionsmitteln bereitgestellten Informationen ausgebildet. Die bereitgestellten Informationen können insbesondere eine räumliche Anordnung der auf die nicht-lichtleitenden Bereiche ausgerichteten LED Pixel in der Emissionsfläche umfassen. Die Steuereinheit ist hierbei vorzugsweise zur selektiven bzw. individuellen Ansteuerung der LED Pixel in Abhängigkeit der erkannten Außenkontur ausgebildet, derart, dass in Hauptabstrahlrichtung auf die Außenkontur und/oder auf einen Bereich radial außerhalb der Außenkontur ausgerichtete LED Pixel bei der Lichteinkopplung wenigstens teilweise, mehr bevorzugt vollständig, mit geringerer Leistung angesteuert oder deaktiviert werden.

Hierdurch kann eine Lichtemission durch das LED Matrixfeld in Richtung auf die Außenkontur des Lichtleiters und/oder auf einen Bereich radial außerhalb der Außenkontur des Lichtleiters reduziert oder vermieden werden, wobei ein unerwünschter Wärmeeintrag in den Lichtleiter und ein effizienterer Betrieb der Vorrichtung während der Lichteinkopplung in den Lichtleiter ermöglicht wird.

In einer weiterhin bevorzugten Ausführungsform ist die Steuerung alternativ oder zusätzlich zur Erkennung einer zusammenhängenden Transmissionsfläche des mit dem Lichtleiteranschluss verbundenen Lichtleiters basierend auf den von den Detektionsmitteln bereitgestellten Informationen, insbesondere hinsichtlich einer räumlichen Anordnung der auf die nicht-lichtleitenden Bereiche ausgerichteten LED Pixel in der Emissionsfläche, ausgebildet. Die Steuereinheit ist hierbei vorzugsweise zur selektiven bzw. individuellen Ansteuerung der LED Pixel in Abhängigkeit der erkannten zusammenhängenden Transmissionsfläche ausgebildet, derart, dass lediglich in Hauptabstrahlrichtung auf die Transmissionsfläche ausgerichteten LED Pixel zur Lichtemission bei der Lichteinkopplung in den Lichtleiter angesteuert werden. Die restlichen LED Pixel werden dann wenigstens mit geringerer Leistung angesteuert und vorzugsweise deaktiviert.

In einer weiterhin bevorzugten Ausführungsform kann die Steuerung alternativ oder zusätzlich zur Erkennung von wenigstens zwei und vorzugsweise einer Mehrzahl von unterschiedlichen Außenkonturen eines Lichtleiters basierend auf den von den Detektionsmitteln bereitgestellten Informationen, insbesondere hinsichtlich einer räumlichen Anordnung der auf die nicht-lichtleitenden Bereiche ausgerichteten LED Pixel in der Emissionsfläche, ausgebildet sein. Hierbei kann die Steuereinheit zur selektiven bzw. individuellen Ansteuerung der LED Pixel in Abhängigkeit der jeweils erkannten Außenkontur gemäß einer hinterlegten bzw. in der Vorrichtung gespeicherten räumlichen Anordnung der LED Pixel zur Lichtemission bei der Lichteinkopplung in den Lichtleiter ausgebildet sein. Hierdurch kann beispielsweise eine durch die jeweilige Außenkontur eines Lichtleiters kodierte Transmissionsfläche des Lichtleiters durch die Steuereinheit dekodiert werden und eine auf die jeweilige Transmissionsfläche abgestimmte Ansteuerung der Emissionsfläche bzw. der LED Pixel der Emissionsfläche erfolgen.

In einer bevorzugten Ausführungsform weist die Beleuchtungsvorrichtung wenigstens einen ersten Kalibriermodus zur Erfassung der auf nicht-lichtleitende Bereiche des Lichtleiters ausgerichteten LED Pixel der Emissionsfläche und einen zweiten Arbeitsbetriebsmodus zur Lichteinkopplung in den Lichtleiter auf. Die Steuereinheit kann hierbei zur sequentiellen und/oder alternierenden Aktivierung des ersten und zweiten Betriebsmodus ausgebildet sein.

Die Steuereinheit kann hierbei derart ausgebildet sein, dass nach Aktivierung der Vorrichtung beispielsweise mittels eines vorgesehenen Bedienelements zunächst der Kalibriermodus aktiviert wird, in welchem eine Erfassung des mit der Vorrichtung verbundenen Lichtleiters mittels der Detektionsmittel erfolgt. Hierbei erfolgt insbesondere die Erfassung der LED Pixel, welche auf nicht-lichtleitende Bereiche des verbundenen Lichtleiters ausgerichtet sind. Anschließend wird der Arbeitsbetriebsmodus aktiviert, in welchem die eigentliche Lichteinkopplung in den Lichtleiter mittels der Beleuchtungsmittel und basierend auf der individuellen Ansteuerung der LED Pixel durch die Steuereinheit erfolgt. Die Kalibrierung erfolgt vorzugsweise innerhalb weniger als einer Sekunde, weiter bevorzugt weniger als 10 Millisekunden.

Eine alternierende Aktivierung des ersten und zweiten Betriebsmodus kann insbesondere eine Aktivierung des Kalibriermodus in vorzugsweise regelmäßigen zeitlichen Abständen, beispielsweise alle 30 Sekunden, umfassen. Hierdurch kann eine kontinuierliche Überprüfung und/oder Korrektur der Lichtleitererkennung durchgeführt werden, beispielsweise falls sich die Positionierung des Lichtleiters am bzw. im Lichtleiteranschluss insbesondere aufgrund von äußeren Einwirkungen ändert.

Die Detektionsmittel sind vorzugsweise zur Detektion von insbesondere entgegen der Hauptabstrahlrichtung der Emissionsfläche auftretender Reflexionsstrahlung, insbesondere durch nicht-lichtleitende Bereiche des Lichtleiters, ausgebildet. Die Detektionsmittel können somit Reflexionsstrahlung erfassen, welchen von den nicht-lichtleitenden Bereichen des Lichtleiters insbesondere in eine Richtung im Wesentlichen entgegen der Hauptabstrahlrichtung reflektiert wird. Bei der Reflexionsstrahlung handelt es sich insbesondere um Lichtstrahlung, welche von wenigstens einem Teil der LED Pixel des LED Matrixfelds emittiert wird und von den nicht-lichtleitenden Bereichen des Lichtleiters rückreflektiert wird.

In einer bevorzugten Ausführungsform sind die Detektionsmittel durch wenigstens einen Teil der LED Pixel des LED Matrixfelds gebildet, welche wenigstens in einem ersten Kalibriermodus der Vorrichtung durch die Steuereinheit als Sensorelemente ansteuerbar ausgebildet sind. Die entsprechenden LED Sensorelemente können hierbei reine Fotodioden sein, welche zur Erfassung einer Lichteinstrahlung auf die jeweilige LED und somit zum Abgriff einer Sensorinformation durch die Steuereinheit ausgebildet sind. In einer besonders bevorzugten Ausführungsform sind die jeweiligen als Sensorelemente ansteuerbaren LED Pixel jedoch derart ausgebildet, dass diese in einem zweiten Arbeitsbetriebsmodus zur Lichtemission ansteuerbar sind.

Zur Ansteuerung der im Arbeitsbetriebsmodus Licht emittierenden LED Pixel als Sensorelement im Kalibriermodus ist die Steuereinheit vorzugsweise derart ausgebildet, dass die jeweiligen LED Pixel im Kalibriermodus nicht mit Strom und/oder Spannung beaufschlagt werden und somit nicht zur Lichtemission angeregt werden. Zusätzlich erfolgt seitens der Steuereinheit ein Abgriff eines Spannungs- und/oder Stromsignales, basierend auf welchem die Steuereinheit auf einen jeweiligen Reflexionslichteinfall auf das jeweilige LED Pixel schließen kann. Hierbei nutzt die Erfindung die Eigenschaft der jeweiligen LED Chips bzw. LED Pixel, dass bei Lichteinfall auf eine ausgeschaltete bzw. nicht durch Strom oder Spannung beaufschlagte LED ein lichteinfallabhängiges Strom- und/oder Spannungssignal an der LED detektiert werden kann.

Die Steuereinheit kann wenigstens einen Mikrokontroller und zugeordnete Speichermittel aufweisen. Die Steuereinheit kann zudem zur Ausführung eines vordefinierten Analyse-Algorithmus ausgebildet sein, welcher durch eine individuelle Ansteuerung der LED Pixel als Sensorelement und/oder als Lichtemissionselement ausgebildet ist.

Die Steuereinheit ist vorzugsweise zur Auswertung und insbesondere zu einem Signalvergleich aller als LED Sensorelement angesteuerten LED Pixel ausgebildet. Durch eine entsprechende Auswertung der abgegriffenen Sensorinformationen bzw. Strom- und/oder Spannungssignale kann eventuell seitlich auf das jeweilige LED Pixel von benachbarten und zur Lichtemission angeregten LED Pixel einfallendes Licht bei der Berechnung bzw. Auswertung gefiltert werden. Anders ausgedrückt kann die Steuereinheit durch Vergleich der abgegriffenen Sensorsignale zwischen als Sensorelement dienenden LED Pixeln unterscheiden, welche lediglich von in der Emissionsfläche benachbarten und zur Emission angeregten LED Pixel teilweise (mit)bestrahlt werden und welche zusätzlicher Reflexionsstrahlung ausgesetzt sind, insbesondere aufgrund im Wesentlichen entgegen der Hauptabstrahlrichtung auftretender Reflexion an nicht-lichtleitenden Bereichen des Lichtleiters. Die Steuereinheit kann sodann letztere als in Hauptabstrahlrichtung auf nicht-lichtleitende Bereiche ausgerichtete LED Pixel der Emissionsfläche identifizieren. Diese können dann als Referenzpunkte auf der Emissionsfläche bzw. im LED Matrixfeld für die individuelle Ansteuerung der Emissionsfläche bei der Lichteinkopplung in den Lichtleiter dienen.

Die Steuereinheit ist vorzugsweise ausgebildet, im ersten Kalibriermodus wenigstens einen ersten Teil der LED Pixel des LED Matrixfelds zur Lichtemission anzusteuern und wenigstens einen zweiten Teil der LED Pixel des LED Matrixfelds als Sensorelemente zur Erkennung von Reflexionslichteinfall auf das jeweilige LED Pixel anzusteuern. Der erste als Emissionselement dienende Teil der LED Pixel kann hierbei eine geringere oder im Wesentlichen gleichgroße Anzahl wie der zweite als Sensorelement dienende Teil der LED Pixel umfassen.

Die Steuereinheit kann zur alternierenden Ansteuerung des ersten und zweiten Teils der LED Pixel als Sensorelement und als Lichtemissionselement ausgebildet sein. Hierdurch kann die Erfassungsgenauigkeit von auf nicht-lichtleitende Bereiche ausgerichtete LED Pixel in der Emissionsfläche weiter verbessert werden.

In einer bevorzugten Ausführungsform ist die Steuereinheit zur Ansteuerung einer Vielzahl von im Wesentlichen gleichmäßig und/oder gemäß einem vordefinierten Kalibrierungsmuster auf der Emissionsfläche verteilten LED Pixeln als Sensorelement ausgebildet. Die als Sensorelement angesteuerten LED Pixel sind hierbei vorzugsweise gleichmäßig zwischen den zur Lichtemission angesteuerten LED Pixeln verteilt angeordnet. Hierdurch wird insbesondere eine optimierte Erfassung von den auf nicht-lichtleitende Bereiche des Lichtleiters ausgerichteten LED Pixeln ermöglicht. In einer weiteren bevorzugten Ausführungsform ist die Steuereinheit im ersten Kalibriermodus zur sequentiellen Ansteuerung der zur Lichtemission angesteuerten LED Pixel in einer von einem Zentrum der Emissionsfläche nach Außen schrittweise radial erweiternder Weise, insbesondere im Wesentlichen konzentrisch oder sternförmig, ausgebildet sein.

Die Steuereinheit kann zur Ansteuerung von wenigstens jedes vierten, vorzugsweise wenigstens jedes dritten der LED Pixel der Emissionsfläche in einer Zeilen- und/oder Spaltenrichtung der Emissionsfläche des LED Matrixfelds als Sensorelement ausgebildet sein. In einer weiteren bevorzugten Ausführungsform sind zwischen zwei in einer Zeilen- und/oder Spaltenrichtung benachbarten und als Emissionselement dienenden LED Pixel wenigstens ein, weiterhin bevorzugt wenigstens zwei, als Sensorelement angesteuerte LED Pixel angeordnet.

In einer alternativen Ausführungsform können die Detektionsmittel eine zwischen der Emissionsfläche und der Transmissionsfläche des Lichtleiters angeordnete Optik, insbesondere einen Strahlenteiler oder einen halbdurchlässigen Spiegel, und eine zugeordnete Sensoreinheit zur Erfassung von durch nicht-leitende Bereiche des Lichtleiters rückreflektierter Strahlung aufweisen. Die zugeordnete Sensoreinheit kann hierbei eine CCD Kamera oder ein Fotodiodenfeld umfassen, welche eine Rückmeldung über Rückreflektionen von nicht-lichtleitenden Bereichen des Lichtleiters zur Steuereinheit ermöglicht.

Der Lichtleiteranschluss ist vorzugsweise zur zentrierten Positionierung wenigstens zweier Lichtleiter mit unterschiedlicher Transmissionsfläche ausgebildet. Der Lichtleiteranschluss weist vorzugsweise eine insbesondere kreisrunde Aufnahme auf, in welche ein endseitig am Lichtleiter angeordneter Stecker eingeführt werden kann. Der Stecker umfasst vorzugsweise eine hülsenartige Ausbildung, welche die endseitig angeordnete Transmissionsfläche zur Einkopplung von Licht in den Lichtleiter umgibt. Der Stecker weist vorzugsweise eine parallel zur Transmissionsfläche angeordnete plane Fläche auf. Der Stecker ist vorzugsweise aus Metall.

Die Vorrichtung weist vorzugsweise keine Kühlmittel zur Kühlung des Lichtleiters und insbesondere des Lichtleitersteckers auf.

In einem weiteren Aspekt betrifft die Erfindung die Verwendung der Beleuchtungsvorrichtung wie vorgehend beschrieben zur Einkopplung von Licht in wenigstens zwei Lichtleiter mit voneinander hinsichtlich Größe und/oder Form abweichenden Transmissionsflächen für die Einkopplung von Licht. Die jeweilige Transmissionsfläche weist vorzugsweise einen Durchmesser zwischen 1,5 und 14mm, mehr bevorzugt zwischen 2 und 10 mm auf. Die jeweilige Transmissionsfläche ist hierbei von einem hülsenartigen Stecker umgeben.

In einem weiteren Aspekt betrifft die Erfindung ein Verfahren zur Einkopplung von Licht in einen Lichtleiter für ein Endoskop aufweisend die Schritte:
- Bereitstellung einer Beleuchtungsvorrichtung mit Beleuchtungsmittel zur Einkopplung von Licht in ein mit der Vorrichtung verbindbaren Lichtleiter und aufweisend ein LED Matrixfeld umfassend eine, eine Emissionsfläche bildende, Vielzahl von LED Pixeln,
- Positionierung einer endseitigen lichtleitenden Transmissionsfläche eines Lichtleiters im Wesentlichen orthogonal zu einer Hauptabstrahlrichtung der Emissionsfläche des LED Matrixfelds, wobei das Verfahren die weiteren Schritte umfasst:
- Detektion von in Hauptabstrahlrichtung auf nicht-lichtleitende Bereiche des Lichtleiters ausgerichtete LED Pixel der Emissionsfläche, und
- Lichteinkopplung in den Lichtleiter unter selektiver Ansteuerung der LED Pixel der Emissionsfläche zur Lichtemission, wobei die auf nicht-lichtleitende Bereiche ausgerichteten LED Pixel wenigstens teilweise mit geringerer Leistung angesteuert werden oder deaktiviert werden.

Zur Vermeidung von Wiederholungen wird auf die zuvor beschriebene erfindungsgemäße Beleuchtungsvorrichtung verwiesen. Die für die Vorrichtung offenbarten Merkmale sollen für das Verfahren offenbart und beanspruchbar sein, sowie umgekehrt.

In einer bevorzugten Ausführungsform umfasst die Detektion eine Ansteuerung eines ersten Teils der LED Pixel der Emissionsfläche zur Lichtemission und eine Ansteuerung eines zweiten Teils der LED Pixel der Emissionsfläche als Sensorelemente zur Erkennung von Reflexionslichteinfall auf das jeweilige LED Pixel.

Die Detektion umfasst vorzugsweise eine variierende Ansteuerung der jeweiligen LED Pixel als Lichtemissionselement oder als Sensorelement. Die variierende Ansteuerung der jeweiligen LED Pixel kann gemäß einer vordefinierten zeitlichen Abfolge und/oder einer räumlichen Anordnung der jeweiligen LED Pixel auf der Emissionsfläche erfolgen. Die Ansteuerung kann eine im Wesentlichen schachbrettartige bzw. -förmige Ansteuerung eines ersten Teils der LED Pixel zur Lichtemission und eines zweiten Teils der LED Pixel als Sensorelement umfassen. Die Ansteuerung kann alternativ oder zusätzlich eine sich von einem Zentrum der Emissionsfläche nach Außen radial erweiternde Ansteuerung eines ersten Teils der LED Pixel zur Lichtemission, insbesondere im Wesentlichen konzentrisch oder sternförmig, umfassen. Die jeweilige Ansteuerung kann gemäß einer vordefinierten Sequenz lediglich einmal, zweimal oder mehrmals durchgeführt werden.

Die Detektion kann zudem eine Erkennung einer Außenkontur des Lichtleiters durch Auswertung eines Sensorabgriffs an den als Sensorelementen angesteuerten LED Pixeln umfassen. Die Erkennung kann hierbei basierend auf den erfassten Signalabgriffen an den LED Pixeln direkt und/oder aufgrund einer seitens der Steuereinheit und basierend auf den Signalabgriffen durchgeführten Interpolation erfolgen. Bei der Lichteinkopplung erfolgt dann eine selektive Ansteuerung der LED Pixel derart, dass in Hauptabstrahlrichtung auf die Außenkontur und/oder auf einen Bereich radial außerhalb der Außenkontur ausgerichtete LED Pixel wenigstens teilweise, vorzugsweise vollständig, mit geringerer Leistung angesteuert oder deaktiviert werden.

Einzelheiten, vorteilhafte Wirkungen und Details der vorliegenden Erfindung werden nachfolgend anhand der rein schematischen, lediglich beispielhaften Zeichnungen erläutert.

Darin zeigen:
- **Fig.1**: eine Beleuchtungsvorrichtung gemäß einer bevorzugten Ausführungsform der Erfindung in Frontalansicht;
- **Fig. 2a**: eine schematische Detailansicht eines Lichtleiters zur Verbindung mit der Beleuchtungsvorrichtung;
- **Fig. 2b**: eine Detailansicht der Vorrichtung ohne verbundenen Lichtleiter;
- **Fig. 3**: eine schematische Draufsicht auf ein LED Matrixfeld der Beleuchtungsmittel der Vorrichtung;
- **Fig. 4**: eine schematische Seitenansicht einer Ankopplung eines Lichtleiters an die Beleuchtungsmittel am Lichtleiteranschluss der Vorrichtung;
- **Fig. 5a,b**: eine bevorzugte Ausführungsform eines Kalibriermodus der Vorrichtung zur Erkennung des Lichtleiters mit im Wesentlich schachbrettförmiger Anordnung der Sensorelemente;
- **Fig. 6a-d**: eine weitere bevorzugte Ausführungsform eines Kalibriermodus der Vorrichtung zur Erkennung des Lichtleiters mit nach Außen radial erweiternder Anordnung der Lichtemissionselemente;
- **Fig. 7a,b**: eine schematische Draufsicht auf die Beleuchtungsmittel bei der unterschiedlichen Ansteuerung von Lichtleiter im Arbeitsbetriebsmodus zur Einkopplung von Licht in den Lichtleiter;
- **Fig. 8a,b**: eine schematische Draufsicht auf die Beleuchtungsmittel bei der unterschiedlichen Ansteuerung von Lichtleitern mit unterschiedlicher Außenkontur;
- **Fig. 9**: eine weitere bevorzugte Ausführungsform der erfindungsgemäßen Vorrichtung, aufweisend zwischen der Emissionsfläche und der Transmissionsfläche angeordnete Optik.

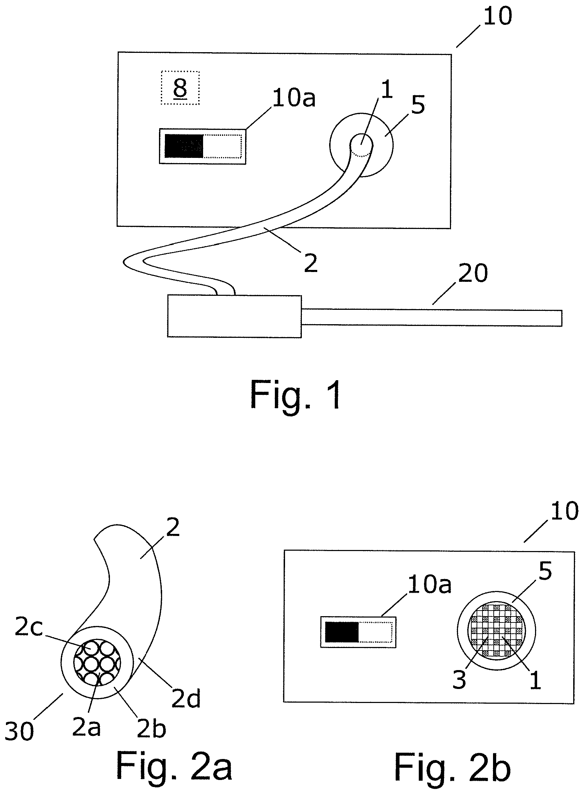

**Fig. 1** zeigt eine Beleuchtungsvorrichtung 10 für ein Endoskop 20 aufweisend Beleuchtungsmittel 1 zur Einkopplung von Licht in einen mit der Vorrichtung über einen Lichtleiteranschluss 5 verbindbaren Lichtleiter 2. Die Vorrichtung kann einen Benutzerschnittstelle 10a aufweisen, welche insbesondere einen Ein-/Ausschalter und/oder eine Steuerung für die Lichtintensität umfassen kann. Die Vorrichtung 10 umfasst weiterhin eine nicht gezeigte Stromversorgung, sowie eine wenigstens mit der Stromversorgung und mit dem Lichtleiteranschluss 5 zugeordneten Beleuchtungsmitteln (vgl. Fig. 2b) verbundene Steuereinheit 8. Der Lichtleiter 2 kann mit dem Endoskop 20 fest verbunden sein oder insbesondere über einen Drehverschluss an das Endoskop auf- bzw. anschraubbar ausgebildet sein.

**Fig. 2a** zeigt schematisch einen endseitigen und für die Verbindung zur Beleuchtungsvorrichtung 10 ausgebildeten Anschluss- bzw. Steckerbereich 30 des Lichtleiters 2. Dieser umfasst eine kreisrunde Transmissionsfläche 2a, welche durch eine Vielzahl von darin angeordneten Faserenden 2c gebildet ist. Die Transmissionsfläche 2a ist von einer nicht-lichtleitenden, vorliegend hülsenartig ausgebildeten Außenkontur 2b umfasst bzw. eingefasst, welche durch eine die optischen Fasern des Lichtleiters umgebende Lichtleiterhülse 2d gebildet wird. Im Anschluss- bzw. Steckerbereich ist die Lichtleiterhülse 2d vorzugsweise plan ausgebildet. Der Steckerbereich 30 und weiterhin bevorzugt die plane Außenkontur 2b ist vorzugsweise aus insbesondere poliertem Metall ausgebildet.

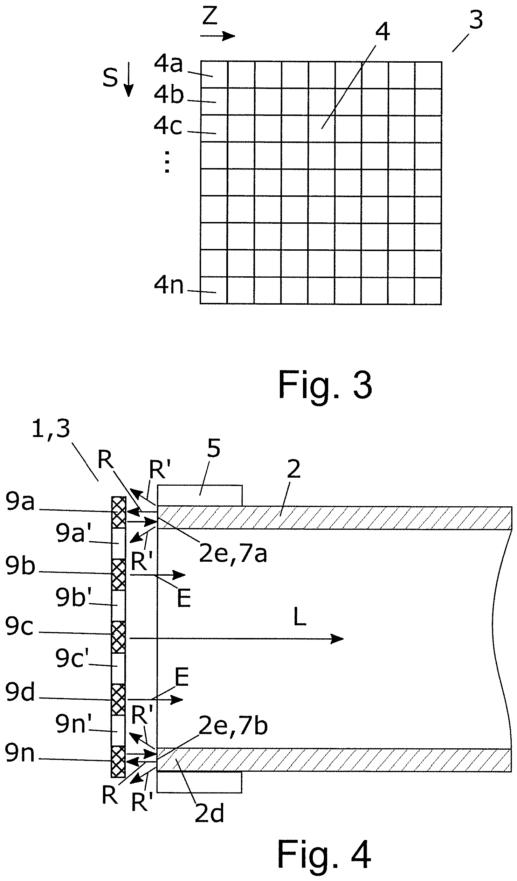

**Fig. 2b** zeigt eine Frontalansicht der Vorrichtung 10 ohne verbundenen Lichtleiter 2. Die Beleuchtungsmittel 1 umfassen hierbei eine zentral bezüglich des Lichtleiteranschlusses 5 angeordnetes LED Matrixfeld 3. **Fig. 3** zeigt eine schematische Detailansicht des LED Matrixfelds 3, welches eine Vielzahl von einzelnen, insbesondere gitterartig bzw. in Zeilen- und Spaltenrichtung Z, S angeordneten LED Pixel 4a,... ,4n aufweist, und welche eine zusammenhängende Emissionsfläche 4 ausbilden.

**Fig. 4** zeigt eine schematische Seitenansicht einer Ankopplung eines Lichtleiters 2 an die Beleuchtungsmittel 1 am Lichtleiteranschluss 5 der Vorrichtung 10. In der verbundenen Anordnung ist der Lichtleiter 2 mit seiner Transmissionsfläche 2a vorzugsweise im Wesentlichen parallel zur Emissionsfläche 4 des LED Matrixfelds 3 angeordnet. Eine Hauptabstrahlrichtung L der Emissionsfläche 4 ist somit im Wesentlichen orthogonal zur Transmissionsfläche 2a angeordnet. Eine endseitige Außenkonturfläche 2e der Lichtleiterhülse 2d ist hierbei ebenfalls vorzugsweise im Wesentlichen parallel zur Emissionsfläche 4 angeordnet.

Die Figur zeigt einen ersten Kalibriermodus der Vorrichtung zur Erfassung der auf nicht-lichtleitende Bereiche 7a,...,7n des Lichtleiters 2 ausgerichteten LED Pixel der Emissionsfläche 4. In dem Kalibriermodus ist die Steuereinheit 8 ausgebildet wenigstens einen ersten Teil der LED Pixel 9a,...,9n des LED Matrixfelds 3 zur Lichtemission E anzusteuern und wenigstens einen zweiten Teil der LED Pixel 9a',...,9n' des LED Matrixfelds 3 als Sensorelemente zur Erkennung von Reflexionslichteinfall R auf das jeweilige LED Pixel anzusteuern.

Zur Vereinfachung ist in der schematischen Zeichnung lediglich eine Lichtemission E der jeweiligen LED Pixel in die Hauptabstrahlrichtung L dargestellt. Wie in Figur 4 gezeigt, tritt eine Reflexion R an den nicht-lichtleitenden Bereichen 7a,b des Lichtleiters 2 auf. Eine hiervon rückreflektierte Strahlung R, welche im Wesentlichen entgegen der Hauptabstrahlrichtung L ausgerichtet ist, kann dann von den jeweils auf die nicht-lichtleitenden Bereichen 7a,b ausgerichteten und als Sensorelemente ausgebildeten LED Pixel 9a',...,9n` und/oder benachbart zu den auf die nicht-lichtleitenden Bereiche 7a,b ausgerichteten und als Emissionselemente dienenden LED Pixel 9a,...,9n erfasst werden. Hierbei wird insbesondere ein an den Sensorelementen 9a',...,9n` durch den Reflexionslichteinfall auftretendes Strom und/oder Spannungssignal durch die Steuereinheit abgegriffen. Die auftretende Reflexion R kann hierbei neben einer direkten Reflexion an den jeweiligen nicht-lichtleitenden Bereichen 7a,7b wenigstens teilweise auch eine diffuse Reflexion bzw. diffuse Reflexionsstrahlung R' umfassen. Unter diffuser Reflexionsstrahlung R' wird eine gestreute Rückstrahlung verstanden, insbesondere aufgrund von möglichen Unebenheiten an der jeweiligen Oberfläche der nicht-lichtleitenden Bereiche 7a,7b,...,7n des Lichtleiters 2.

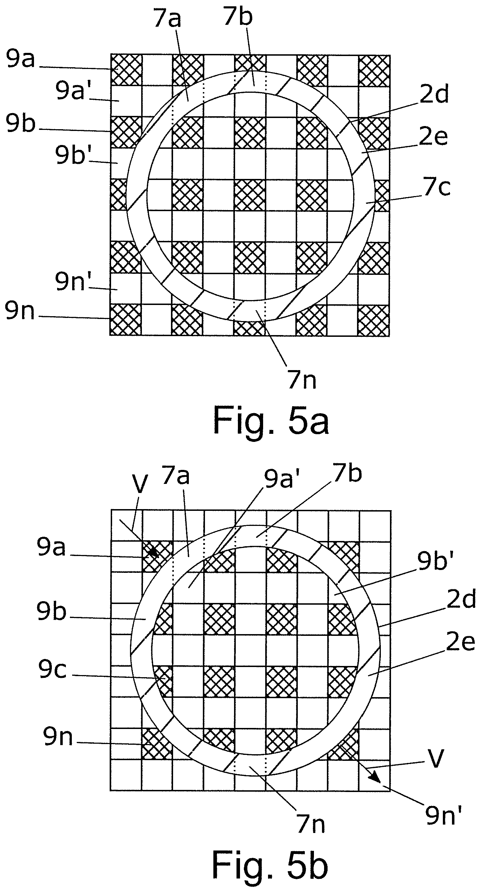

Die Steuereinheit 8 ist vorzugsweise derart ausgebildet, dass diese die jeweiligen LED Pixel 4a,...4n variierend und/oder alternierend als Emissionselemente 9a,...,9n und als Sensorelemente 9a',...9n' ansteuert. Eine derartige Ausbildung ist beispielhaft in Fig. 5a und 5b gezeigt. Die Steuereinheit 8 ist hierbei ausgebildet, die ersten als Emissionselement angesteuerten LED Pixel 9a,...,9n und die zweiten als Sensorelement angesteuerten LED Pixel 9a',...9n' gemäß einem vordefinierten Kalibrierungsmuster anzusteuern, vorliegend beispielhaft gemäß einer schachbrettförmigen Anordnung. Hierbei kann beispielsweise jede zweite LED 4a,...,4n der Emissionsfläche 4 in Zeilenrichtung Z und/oder Spaltenrichtung S (vgl. Fig. 3) als Emissionselement 9a,...,9n angesteuert werden, um eine Lichtemission für die Erfassung der nicht-lichtleitenden Bereiche des verbundenen Lichtleiters 2 bereitzustellen. Die restlichen LED Pixel können als Sensorelement 9a',...9n' angesteuert werden, zur Erfassung von Reflexion an nicht-lichtleitenden Bereichen 7a,...,7n des Lichtleiters 2, insbesondere an der die Transmissionsfläche 2a umgebende Außenkonturfläche 2e der Lichtleiterhülse 2d. Die nicht lichtleitenden Bereiche, 7a, ... ,7n sind in Fig. 5a, 5b lediglich beispielhaft und schematisch dargestellt. Die nicht-lichtleitenden Bereiche 7a,...,7n bilden vorzugsweise die Außenkonturfläche 2e des Lichtleiters 2.

Nach erfolgter Emission und Detektion in der in Fig. 5a gezeigten Anordnung kann eine variierende Ansteuerung der einzelnen LED Pixel 4a,...4n erfolgen, wie in Fig. 5b gezeigt. Hierbei wird die schachbrettförmige Ansteuerung der einzelnen LED Pixel parallel oder diagonal um einen Versatz V gegenüber der Ausbildung in Fig. 5a versetzt bzw. verschoben. Dies bedeutet, dass nunmehr zuvor als Sensorelement 9a',...9n' angesteuert LED Pixel als Emissionselement 9a,...9n angesteuert werden und umgekehrt. Dabei erfolgt eine erneute Erkennung der jeweils an den Sensorelementen 9a',...9n' durch den Reflexionslichteinfall R auftretenden Strom und/oder Spannungssignale, durch die Steuereinheit 8.

Die Steuereinheit 8 kann hierbei zu einem Signalvergleich aller als LED Sensorelement angesteuerten LED Pixel 9a',... 9n' ausgebildet sein. Hierbei kann die Steuereinheit 8 vorzugsweise durch Vergleich der abgegriffenen Sensorsignale zwischen als Sensorelement dienenden LED Pixeln unterscheiden, welche lediglich von in der Emissionsfläche 4 benachbart angeordneten Emissionselementen 9a,...,9n (mit)bestrahlt werden und welche zusätzlicher Reflexionsstrahlung R ausgesetzt sind, insbesondere aufgrund im Wesentlichen entgegen der Hauptabstrahlrichtung L auftretender Reflexion R an nicht-lichtleitenden Bereichen 7a,...7n des Lichtleiters 2. Die Steuereinheit 8 kann sodann letztere als in Hauptabstrahlrichtung L auf nicht-lichtleitende Bereiche ausgerichtete LED Pixel der Emissionsfläche 4 identifizieren. Diese können dann als Referenzpunkte auf der Emissionsfläche 4 bzw. im LED Matrixfeld 3 für die individuelle Ansteuerung der Emissionsfläche 4 bei der Lichteinkopplung in den Lichtleiter 2 dienen.

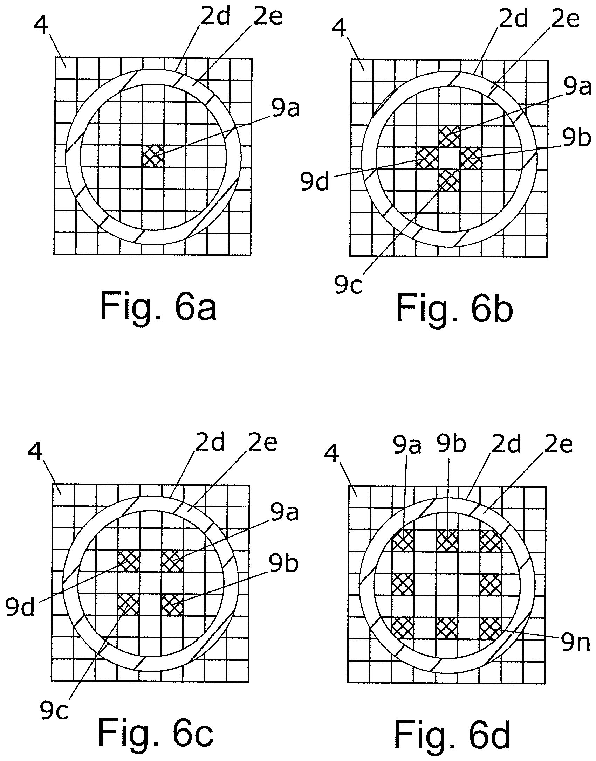

**Fig. 6a-6d** zeigen eine alternative Ausführungsform, in welcher die LED Pixel 4a,...,4n derart im Kalibrierungsmodus angesteuert werden, dass eine von einem Zentrum der Emissionsfläche schrittweise nach Außen und sich vorzugsweise radial erweiternde Anordnung der jeweils zur Emission angesteuerten LED Pixel 9a,...,9n ergibt. Die restlichen LED Pixel können als Sensorelement 9a',... ,9n` angesteuert werden.

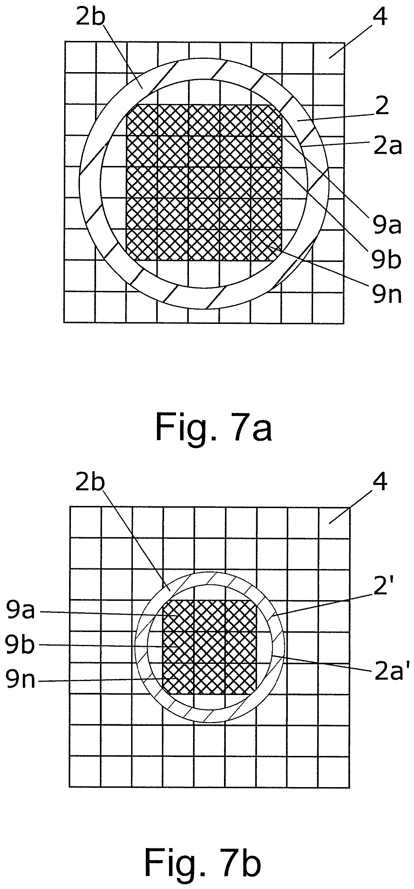

**Fig. 7a,7b** eine schematische Draufsicht auf die Beleuchtungsmittel 1 bei der unterschiedlichen Ansteuerung von Lichtleitern 2,2` mit unterschiedlicher Transmissionsfläche 2a,2a` im Arbeitsbetriebsmodus. Der Arbeitsbetriebsmodus der Vorrichtung 10 entspricht dem Betrieb zur Einkopplung von Licht in den jeweils mit der Vorrichtung verbundenen Lichtleiter 2,2`. Durch die zuvor durchgeführte Kalibrierung erkennt die Steuereinheit 8 LED Pixel der Emissionsfläche 4, welche auf nicht-lichtleitende Bereiche des Lichtleiters 2 ausgerichtet sind. Diese werden im Arbeitsbetriebsmodus vorzugsweise mit weniger Leistung angesteuert oder weiter bevorzugt deaktiviert. Die restlichen LED Pixel 9a,...,9n werden zur Lichtemission mit einer vordefinierten und optional durch den Benutzer einstellbaren Leistung angesteuert.

Die Steuereinheit 8 erkennt weiterhin bevorzugt im Kalibriermodus eine Außenkontur 2b des mit der Vorrichtung verbundenen Lichtleiters 2 und steuert im Arbeitsbetriebsmodus die in Hauptabstrahlrichtung L auf die Außenkontur 2b und/oder auf einen Bereich radial außerhalb der Außenkontur 2b ausgerichteten LED Pixel bei der Lichteinkopplung wenigstens teilweise mit geringerer Leistung an oder deaktiviert diese.

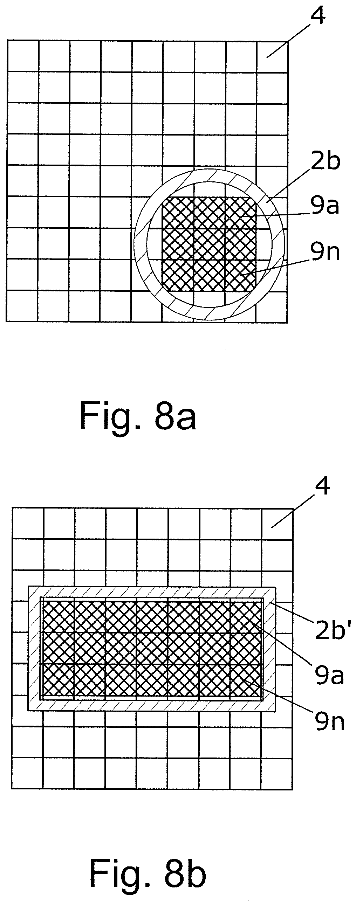

**Fig. 8a** und **8b** zeigen beispielhaft und schematisch eine Erkennung der jeweiligen Außenkontur 2b eines verbundenen Lichtleiters. Wie in **Fig. 8a** gezeigt, kann die Steuereinheit 8 ausgebildet sein, ein Verschieben bzw. einen Versatz des Lichtleiters 2 bezüglich der Emissionsfläche 4, beispielsweise bei Außeneinwirkung, detektieren. Dies kann durch alternierende Aktivierung des zuvor beschriebenen Kalibriermodus und des Arbeitsbetriebsmodus erfolgen. Insbesondere kann der Kalibriermodus in regelmäßigen Abständen während des Arbeitsbetriebsmodus aktiviert werden. Die Ansteuerung der Emissionsfläche 4 kann im folgenden Arbeitsbetriebsmodus entsprechend angepasst werden.

**Fig. 8b** zeigt lediglich beispielhaft die Erkennung einer variierenden Außenkontur 2b' eines Lichtleiters 2, welche abweichend von der zuvor gezeigten Außenkontur eine vieleckige Kontur aufweist. Die Steuerung kann hierbei optional zur Erkennung von wenigstens zwei und vorzugsweise einer Mehrzahl von unterschiedlichen Außenkonturen 2b,2b` eines Lichtleiters 2 basierend auf den von den Detektionsmitteln bereitgestellten Informationen, insbesondere hinsichtlich einer räumlichen Anordnung der auf die nicht-lichtleitenden Bereiche ausgerichteten LED Pixel in der Emissionsfläche, ausgebildet sein. Hierbei kann die Steuereinheit dann zur individuellen Ansteuerung der LED Pixel in Abhängigkeit der jeweils erkannten Außenkontur gemäß einer hinterlegten bzw. in der Vorrichtung gespeicherten räumlichen Anordnung der LED Pixel zur Lichtemission bei der Lichteinkopplung in den Lichtleiter ausgebildet sein.

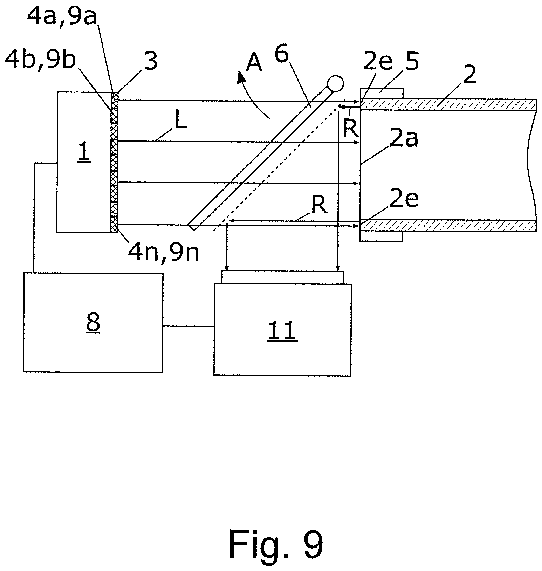

**Fig. 9** zeigt eine alternative Ausführungsform der erfindungsgemäßen Vorrichtung 10. Hierbei umfassen die Detektionsmittel eine zwischen der Emissionsfläche 4 und der Transmissionsfläche 2a des Lichtleiters 2 angeordnete Optik 6, insbesondere einen Strahlenteiler oder halbdurchlässigen Spiegel sowie eine zugeordnete Sensoreinheit 11 zur Erfassung von durch nicht-lichtleitende Bereichen 7a,...,7n des Lichtleiters 2 rückreflektierter Strahlung R. Die Sensoreinheit 11 kann hierbei eine CCD Kamera oder ein Fotodiodenfeld umfassen.

Im Kalibriermodus wird über die Optik, beispielhaft den halbdurchlässigen Spiegel, ein Teil des emittieren Lichts, welcher von den nicht-lichtleitenden Bereichen des Lichtleiters 2 zurückreflektiert wird, von der zugeordneten Sensoreinheit 11 erfasst. Hierbei kann insbesondere eine Relativpositionserfassung der jeweiligen Rückstrahlung R erfolgen. Basierend auf dieser Erfassung kann im Arbeitsbetriebsmodus eine entsprechende Ansteuerung der jeweiligen LED Pixel 4a,...,4n erfolgen, wie zuvor für die anderen Ausführungsbeispiele beschrieben. Im Arbeitsbetriebsmodus kann die Optik 6 zudem aus dem Emissionsbereich der Beleuchtungsmittel 1 entfernt werden, beispielsweise durch Verschwenken (Richtung A).

### Bezugszeichenliste

- 1: Beleuchtungsmittel
- 2: Lichtleiter
- 2a: Transmissionsfläche
- 2b: Außenkontur
- 2c: lichtleitende Fasern
- 2d: Lichtleiterhülse
- 2e: Außenkonturfläche
- 3: Matrixfeld
- 4: Emissionsfläche
- 4a,..,4n: LED Pixel
- 5: Lichtleiteranschluss
- 6: Optik
- 7a,...,7n: nicht-lichtleitende Teile des Lichtleiters
- 8: Steuereinheit
- 9a,...,9n: Emissionselement
- 9a',...,9n': Sensorelement
- 10: Vorrichtung
- 10a: Benutzerschnittstelle
- 11: Sensoreinheit
- 20: Endoskop
- 30: Steckerbereich

- A: Schwenkrichtung
- E: Emissionsstrahlung
- L: Hauptabstrahlrichtung
- R,R': Reflexionsstrahlung
- S: Spaltenrichtung
- V: Versatz
- Z: Zeilenrichtung

## Patentansprüche

1. Beleuchtungsvorrichtung (10) für ein Endoskop (20) aufweisend Beleuchtungsmittel (1) zur Einkopplung von Licht in einen mit der Vorrichtung verbindbaren Lichtleiter (2) und aufweisend ein LED Matrixfeld (3) umfassend eine, eine Emissionsfläche (4) bildende, Vielzahl von LED Pixeln (4a,...,4n), und
einen den Beleuchtungsmitteln (1) zugeordneten Lichtleiteranschluss (5) zur Positionierung einer endseitig am Lichtleiter angeordneten lichtleitenden Transmissionsfläche (2a) im Wesentlichen orthogonal zu einer Hauptabstrahlrichtung (L) der Emissionsfläche (4) des LED Matrixfelds (3),
**dadurch gekennzeichnet, dass** die Vorrichtung Detektionsmittel aufweist, welche zur Erfassung von in Hauptabstrahlrichtung (L) auf nicht-lichtleitende Bereiche (7a,...,7n) des Lichtleiters (2) ausgerichteten LED Pixeln der Emissionsfläche (4) ausgebildet sind, und dass die Vorrichtung (10) eine Steuereinheit (8) aufweist, welche zur selektiven Ansteuerung der LED Pixel (4a,...,4n) des LED Matrixfelds (3) für die Lichteinkopplung in den Lichtleiter (2) ausgebildet ist, derart, dass die in Hauptabstrahlrichtung (L) auf die nicht-leitenden Bereiche (7a,...,7n) des Lichtleiters (2) ausgerichteten LED Pixel wenigstens teilweise mit geringerer Leistung angesteuert oder deaktiviert werden.

2. Beleuchtungsvorrichtung nach Anspruch 1, **dadurch gekennzeichnet, dass** die Steuereinheit (8) zur Erkennung einer die Transmissionsfläche (2a) umgebenden nicht-lichtleitenden Außenkontur (2b) des Lichtleiters (2) basierend auf von den Detektionsmitteln bereitgestellten Informationen ausgebildet ist, und dass die Steuereinheit (8) zur selektiven Ansteuerung der LED Pixel (4a,...,4n) in Abhängigkeit der erkannten Außenkontur (2b) ausgebildet ist, derart, dass in Hauptabstrahlrichtung (L) auf die Außenkontur (2b) und/oder auf einen Bereich radial außerhalb der Außenkontur (2b) ausgerichtete LED Pixel (4a,...,4n) bei der Lichteinkopplung wenigstens teilweise mit geringerer Leistung angesteuert oder deaktiviert werden.

3. Beleuchtungsvorrichtung nach einem der Ansprüche 1 oder 2, **dadurch gekennzeichnet, dass** die Beleuchtungsvorrichtung (10) wenigstens einen ersten Kalibriermodus zur Erfassung der auf nicht-lichtleitende Bereiche (7a,...,7n) des Lichtleiters (2) ausgerichteten LED Pixel der Emissionsfläche (4) und einen zweiten Arbeitsbetriebsmodus zur Lichteinkopplung in den Lichtleiter (2) aufweist, und dass die Steuereinheit (8) zur sequentiellen und/oder alternierenden Aktivierung des ersten und zweiten Betriebsmodus ausgebildet ist.

4. Beleuchtungsvorrichtung nach einem der Ansprüche 1 bis 3, **dadurch gekennzeichnet, dass** die Detektionsmittel zur Detektion von insbesondere entgegen der Hauptabstrahlrichtung (L) der Emissionsfläche (4) auftretender Reflexionsstrahlung (R), insbesondere durch nicht-lichtleitende Bereiche (7a,...,7n) des Lichtleiters (2), ausgebildet sind.

5. Beleuchtungsvorrichtung nach einem der vorhergehenden Ansprüche, **dadurch gekennzeichnet, dass** die Detektionsmittel durch wenigstens einen Teil der LED Pixel (4a,...,4n) des LED Matrixfelds (3) gebildet sind, welche wenigstens in einem ersten Kalibriermodus der Vorrichtung (10) durch die Steuereinheit (8) als Sensorelemente ansteuerbar und wenigstens in einem zweiten Arbeitsbetriebsmodus zur Lichtemission ansteuerbar ausgebildet sind.

6. Beleuchtungsvorrichtung nach Anspruch 5, **dadurch gekennzeichnet, dass** die Steuereinheit (8) ausgebildet ist, im ersten Kalibiermodus wenigstens einen ersten Teil der LED Pixel des LED Matrixfelds (3) zur Lichtemission anzusteuern und wenigstens einen zweiten Teil der LED Pixel des LED Matrixfelds (3) als Sensorelemente zur Erkennung von Reflexionslichteinfall auf das jeweilige LED Pixel anzusteuern.

7. Beleuchtungsvorrichtung nach Anspruch 6, **dadurch gekennzeichnet, dass** die Steuereinheit (8) zur alternierenden Ansteuerung des ersten und zweiten Teils der LED Pixel (4a,...,4n) als Sensorelement und als Lichtemissionselement ausgebildet ist.

8. Beleuchtungsvorrichtung nach einem der Ansprüche 5 bis 7, **dadurch gekennzeichnet, dass** die Steuereinheit (8) zur Ansteuerung einer Vielzahl von im Wesentlichen gleichmäßig und/oder gemäß einem vordefinierten Kalibrierungsmuster auf der Emissionsfläche (4) verteilten LED Pixeln (4a,...,4n) als Sensorelement ausgebildet ist.

9. Beleuchtungsvorrichtung nach einem der Ansprüche 5 bis 8, **dadurch gekennzeichnet, dass** die Steuereinheit (8) zur Ansteuerung von wenigstens jedes vierten, vorzugsweise wenigstens jedes dritten der LED Pixel der Emissionsfläche (4) in einer Zeilen- und/oder Spaltenrichtung (Z,S) der Emissionsfläche (4) des LED Matrixfelds (3) als Sensorelement ausgebildet ist.

10. Beleuchtungsvorrichtung nach einem der Ansprüche 5 bis 9, **dadurch gekennzeichnet, dass** die Steuereinheit (8) im ersten Kalibriermodus zur sequentiellen Ansteuerung der zur Lichtemission angesteuerten LED Pixel in einer von einem Zentrum der Emissionsfläche nach Außen schrittweise radial erweiternden Weise, insbesondere im Wesentlichen konzentrisch oder sternförmig, ausgebildet ist.

11. Beleuchtungsvorrichtung nach einem der vorhergehenden Ansprüche 1 bis 4, **dadurch gekennzeichnet, dass** die Detektionsmittel eine zwischen der Emissionsfläche (4) und der Transmissionsfläche (2a) des Lichtleiters (2) angeordnete Optik, insbesondere einen Strahlenteiler oder halbdurchlässigen Spiegel, und eine zugeordnete Sensoreinheit (11) zur Erfassung von durch nicht-leitende Bereiche (7a,...,7n) des Lichtleiters (2) rückreflektierter Strahlung (R) aufweisen, insbesondere eine CCD Kamera oder ein Fotodiodenfeld.

12. Beleuchtungsvorrichtung nach einem der vorhergehenden Ansprüche, **dadurch gekennzeichnet, dass** das LED Matrixfeld (3) vorzugsweise zwischen 500 und 4500, mehr bevorzugt zwischen 1000 und 4500 LED Pixel (4a,...,4n) aufweist, welche vorzugsweise aus weißlichtemittierenden LED Chips gebildet sind.

13. Beleuchtungsvorrichtung nach einem der vorhergehenden Ansprüche, **dadurch gekennzeichnet, dass** der Lichtleiteranschluss (5) zur vorzugsweise zentrierten Positionierung wenigstens zweier Lichtleiter (2,2`) mit unterschiedlicher Transmissionsfläche ausgebildet ist.

14. Verfahren zur Einkopplung von Licht in einen Lichtleiter für ein Endoskop aufweisend die Schritte:
- Bereitstellung einer Beleuchtungsvorrichtung (10) mit Beleuchtungsmittel (1) zur Einkopplung von Licht in einen mit der Vorrichtung verbindbaren Lichtleiter (2) und aufweisend ein LED Matrixfeld (3) umfassend eine, eine Emissionsfläche (4) bildende, Vielzahl von LED Pixeln (4a,...,4n),
- Positionierung einer endseitigen lichtleitenden Transmissionsfläche (2a) eines Lichtleiters (2) im Wesentlichen orthogonal zu einer Hauptabstrahlrichtung (L) der Emissionsfläche (4) des LED Matrixfelds (3), **dadurch gekennzeichnet, dass** das Verfahren die weiteren Schritte umfasst:
- Detektion von in Hauptabstrahlrichtung auf nicht-lichtleitende Bereiche (7a,...,7n) des Lichtleiters (2) ausgerichtete LED Pixel der Emissionsfläche (4), und
- Lichteinkopplung in den Lichtleiter (2) unter selektiver Ansteuerung der LED Pixel der Emissionsfläche (4) zur Lichtemission, wobei die auf nicht-lichtleitende Bereiche (7a,...,7n) ausgerichteten LED Pixel wenigstens teilweise mit geringerer Leistung angesteuert werden oder deaktiviert werden.

15. Verfahren nach Anspruch 14, **dadurch gekennzeichnet, dass** die Detektion eine Ansteuerung eines ersten Teils der LED Pixel der Emissionsfläche zur Lichtemission und eine Ansteuerung eines zweiten Teils der LED Pixel der Emissionsfläche als Sensorelemente zur Erkennung von Reflexionslichteinfall auf das jeweilige LED Pixel umfasst.

16. Verfahren nach Anspruch 15, **dadurch gekennzeichnet, dass** die Detektion eine variierende Ansteuerung der jeweiligen LED Pixel als Lichtemissionselement (9a,...,9n) oder als Sensorelement (9a',...,9n`) umfasst, vorzugsweise gemäß einer vordefinierten zeitlichen Abfolge und/oder einer räumlichen Anordnung der jeweiligen LED Pixel auf der Emissionsfläche.

17. Verfahren nach Anspruch 15 oder 16, **dadurch gekennzeichnet, dass** die Ansteuerung eine im Wesentlichen schachbrettartige Ansteuerung eines ersten Teils der LED Pixel zur Lichtemission und eines zweiten Teils der LED Pixel als Sensorelement umfasst.

18. Verfahren nach einem der Ansprüche 15 bis 17, **dadurch gekennzeichnet, dass** die Ansteuerung eine sich von einem Zentrum der Emissionsfläche nach Außen radial erweiternde Ansteuerung eines ersten Teils der LED Pixel zur Lichtemission, insbesondere im Wesentlichen konzentrisch oder sternförmig, umfasst.

19. Verfahren nach einem der Ansprüche 15 bis 18, **dadurch gekennzeichnet, dass** die Detektion eine Erkennung einer Außenkontur (2b) des Lichtleiters (2) durch Auswertung eines Sensorabgriffs an den als Sensorelementen angesteuerten LED Pixeln umfasst, wobei bei der Lichteinkopplung die LED Pixel selektiv derart angesteuert werden, dass in Hauptabstrahlrichtung (L) auf die Außenkontur (2b) und/oder auf einen Bereich radial außerhalb der Außenkontur (2b) ausgerichtete LED Pixel wenigstens teilweise mit geringerer Leistung angesteuert oder deaktiviert werden.

20. Verwendung der Beleuchtungsvorrichtung nach einem der Ansprüche 1 bis 13 zur Einkopplung von Licht in wenigstens zwei Lichtleiter (2,2`) mit voneinander hinsichtlich Größe und/oder Form abweichenden Transmissionsflächen (2a,2a`).

## Claims

1. An illumination device (10) for an endoscope (20), the device comprising illumination means (1) for coupling light into a light guide (2) connectable to the device, the illumination means comprising an LED matrix array (3) comprising a plurality of LED pixels (4a, ..., 4n) forming an emission area (4), and
a light guide port (5) associated with the illumination means (1) for positioning a light-guiding transmission area (2a), which is disposed at an end of the light guide, essentially orthogonally to a main emission direction (L) of the emission area (4) of the LED matrix array (3),
**characterized in that** the device comprises detection means which are configured to detect LED pixels of the emission area (4) that are directed at non-light-guiding portions (7a, ..., 7n) of the light guide (2) in the main emission direction (L), and that the device (10) has a control unit (8) configured to selectively operate the LED pixels (4a, ..., 4n) of the LED matrix array (3) for coupling light into the light guide (2) in such a manner that at least some of the LED pixels directed at the non-guiding portions (7a, ..., 7n) of the light guide (2) in the main emission direction (L) are operated at lower power or deactivated.

2. The illumination device according to claim 1, **characterized in that** the control unit (8) is configured to detect a non-light-guiding outer contour (2b) of the light guide (2) surrounding the transmission area (2a) based on information provided by the detection means, and that the control unit (8) is configured to selectively operate the LED pixels (4a, ..., 4n) as a function of the detected outer contour (2b) in such a manner that at least some of the LED pixels (4a, ..., 4n) directed at the outer contour (2b) and/or at an area radially outside of the outer contour (2b) in the main emission direction (L) are operated at lower power or deactivated when light is being coupled in.

3. The illumination device according to any one of claims 1 or 2, **characterized in that** the illumination device (10) comprises at least a first calibrating mode for detecting the LED pixels of the emission area (4) that are directed at non-light-guiding portions (7a, ..., 7n) of the light guide (2) and a second work operating mode for coupling light into the light guide (2), and that the control unit (8) is configured to sequentially and/or alternately activate the first and the second operating mode.

4. The illumination device according to any one of claims 1 to 3, **characterized in that** the detection means are configured to detect reflected radiation (R) occurring in particular in a direction opposite to the main emission direction (L) of the emission area (4), in particular radiation (R) reflected by non-light-guiding portions (7a, ..., 7n) of the light guide (2).

5. The illumination device according to any one of the preceding claims, **characterized in that** the detection means are formed by at least some of the LED pixels (4a, ..., 4n) of the LED matrix array (3) which are configured to be operated as sensor elements by the control unit (8) at least in a first calibrating mode of the device (10) and to be operated for light emission at least in a second work operating mode.

6. The illumination device according to claim 5, **characterized in that** the control unit (8) is configured to operate at least a first part of the LED pixels of the LED matrix array (3) for light emission in the first calibrating mode and to operate at least a second part of the LED pixels of the LED matrix array (3) as sensor elements for detecting reflected light incident on the respective LED pixel.

7. The illumination device according to claim 6, **characterized in that** the control unit (8) is configured to alternately operate the first and the second part of the LED pixels (4a, ..., 4n) as sensor elements and as light emission elements.

8. The illumination device according to any one of claims 5 to 7, **characterized in that** the control unit (8) is configured to operate a plurality of LED pixels (4a, ..., 4n) distributed on the emission area (4) essentially uniformly and/or according to a pre-defined calibrating pattern as sensor elements.

9. The illumination device according to any one of claims 5 to 8, **characterized in that** the control unit (8) is configured to operate at least every fourth, preferably every third LED pixel of the emission area (4) in a row direction (Z) and/or a column direction (S) of the emission area (4) of the LED matrix array (3) as a sensor element.

10. The illumination device according to any one of claims 5 to 9, **characterized in that** the control unit (8) in the first calibrating mode is configured to sequentially operate the LED pixels operated for light emission in a manner gradually widening radially outward from a center of the emission area, in particular essentially concentrically or in a star shape.

11. The illumination device according to any one of preceding claims 1 to 4, **characterized in that** the detection means comprise optics, in particular a beam splitter or a semi-transparent mirror, disposed between the emission area (4) and the transmission area (2a) of the light guide (2), and an associated sensor unit (11), in particular a CCD camera or a photodiode array, for detecting radiation (R) reflected back by non-guiding portions (7a, ..., 7n) of the light guide (2).

12. The illumination device according to any one of the preceding claims, **characterized in that** the LED matrix array (3) preferably comprises between 500 and 4500, more preferably between 1000 and 4500 LED pixels (4a, ..., 4n), which are preferably formed by white light emitting LED chips.

13. The illumination device according to any one of the preceding claims, **characterized in that** the light guide port (5) is configured to position, preferably center, at least two light guides (2, 2') having different transmission areas.

14. A method for coupling light into a light guide for an endoscope, the method comprising the following steps:
- providing an illumination device (10) comprising illumination means (1) for coupling light into a light guide (2) connectable to the device, the illumination means comprising an LED matrix array (3) comprising a plurality of LED pixels (4a, ..., 4n) forming an emission area (4),
- positioning a light-guiding transmission area (2a), which is disposed at an end of a light guide (2), essentially orthogonally to a main emission direction (L) of the emission area (4) of the LED matrix array (3), **characterized in that** the method further comprises the following steps:
- detecting LED pixels of the emission area (4) that are directed at non-light-guiding portions (7a, ..., 7n) of the light guide (2) in the main emission direction, and
- coupling light into the light guide (2), with the LED pixels of the emission area (4) being selectively operated for light emission, wherein at least some of the LED pixels directed at non-light-guiding portions (7a, ..., 7n) are operated at lower power or are deactivated.

15. The method according to claim 14, **characterized in that** the detection comprises operating a first part of the LED pixels of the emission area for light emission and operating a second part of the LED pixels of the emission area as sensor elements for detecting reflected light incident on the respective LED pixel.

16. The method according to claim 15, **characterized in that** the detection comprises operating the respective LED pixels in a varying manner as light emission elements (9a, ..., 9n) or as sensor elements (9a', ..., 9n'), preferably according to a predefined chronological order and/or a spatial arrangement of the respective LED pixels on the emission area.

17. The method according to claim 15 or 16, **characterized in that** the operation comprises operating a first part of the LED pixels for light emission and operating a second part of the LED pixels as sensor elements in an essentially chessboard-like manner.

18. The method according to any one of claims 15 to 17, **characterized in that** the operation comprises operating a first part of the LED pixels for light emission in a manner widening radially outward from a center of the emission area, in particular essentially concentrically or in a star shape.

19. The method according to any one of claims 15 to 18, **characterized in that** the detection comprises detecting an outer contour (2b) of the light guide (2) by evaluating sensor data acquired by the LED pixels operated as sensor elements, wherein during the coupling in of light the LED pixels are selectively operated in such a manner that at least some of the LED pixels directed in the main emission direction (L) at the outer contour (2b) and/or at an area radially outside of the outer contour (2b) are operated at lower power or deactivated.

20. A use of the illumination device according to any one of claims 1 to 13 for coupling light into at least two light guides (2, 2') having transmission areas (2a, 2a') differing in size and/or shape.

## Revendications

1. Dispositif d'illumination (10) pour un endoscope (20), le dispositif comprenant un moyen d'illumination (1) pour coupler la lumière dans un guide de lumière (2) connectable au dispositif, le moyen d'illumination comprenant un réseau matriciel LED (3) comprenant une pluralité de pixels LED (4a, ..., 4n) formant une surface d'émission (4), et
une connexion (5) de guide de lumière associée aux moyens d'illumination (1) pour positionner une surface (2a) de transmission de lumière, qui guide la lumière et est disposée à une extrémité du guide de lumière, essentiellement orthogonalement à une direction d'émission principale (L) de la surface d'émission (4) du réseau matriciel LED (3),
**caractérisé en ce que** le dispositif comprend des moyens de détection qui sont configurés pour détecter des pixels LED de la surface d'émission (4) qui sont dirigés vers des parties (7a, ..., 7n) non guidant la lumière du guide de lumière (2) dans la direction d'émission principale (L), et **en ce que** le dispositif (10) a une unité de commande (8) configurée pour commander sélectivement les pixels LED (4a, ..., 4n) du réseau matriciel LED (3) pour coupler la lumière dans le guide de lumière (2) de telle sorte qu'au moins certains des pixels LED dirigés vers les parties (7a, ..., 7n) non guidant du guide de lumière (2) dans la direction d'émission principale (L) sont commandés à une puissance inférieure ou désactivés.

2. Dispositif d'illumination selon la revendication 1, **caractérisé en ce que** l'unité de commande (8) est configurée pour détecter un contour extérieur (2b) non guidant la lumière du guide de lumière (2) entourant la surface de transmission (2a) sur la base des informations fournies par le moyen de détection, et **en ce que** l'unité de commande (8) est configurée pour commander sélectivement les pixels LED (4a, ..., 4n) en fonction du contour extérieur (2b) détecté de telle sorte qu'au moins une certains des pixels LED (4a, ..., 4n) dirigés vers le contour extérieur (2b) et/ou vers une zone située radialement à l'extérieur du contour extérieur (2b) dans la direction d'émission principale (L) sont commandés à une puissance inférieure ou désactivés lorsque la lumière est couplée à l'intérieur.

3. Dispositif d'illumination selon l'une quelconque des revendications 1 ou la revendication 2, **caractérisé en ce que** le dispositif d'illumination (10) comprend au moins un premier mode de calibrage pour détecter les pixels LED de la surface d'émission (4) qui sont dirigés vers des parties (7a, ..., 7n) non guidant de la lumière du guide de lumière (2) et un second mode de fonctionnement de travail pour coupler la lumière dans le guide de lumière (2), et **en ce que** l'unité de commande (8) est configurée pour activer séquentiellement et/ou alternativement le premier et le second mode de fonctionnement.

4. Dispositif d'illumination selon l'une quelconque des revendications 1 à 3, **caractérisé en ce que** les moyens de détection sont configurés pour détecter un rayonnement réfléchi (R) se produisant notamment dans une direction opposée à la direction d'émission principale (L) de la surface d'émission (4), notamment un rayonnement (R) réfléchi par des parties (7a, ..., 7n) non guidant du guide de lumière (2).

5. Dispositif d'illumination selon l'une quelconque des revendications précédentes, **caractérisé en ce que** les moyens de détection sont formés par au moins certains des pixels LED (4a, ..., 4n) du réseau matriciel LED (3) qui sont configurés pour être commandés en tant qu'éléments de capteur par l'unité de commande (8) au moins dans un premier mode de calibrage du dispositif (10) et pour être commandés pour l'émission de lumière au moins dans un second mode d'exploitation de travail.

6. Dispositif d'illumination selon la revendication 5, **caractérisé en ce que** l'unité de commande (8) est configurée pour commander au moins une première partie des pixels LED du réseau matriciel LED (3) pour l'émission de lumière dans le premier mode de calibrage et pour commander au moins une deuxième partie des pixels LED du réseau matriciel LED (3) en tant qu'éléments de capteur pour détecter la lumière réfléchie incidente sur le pixel LED respectif.

7. Dispositif d'illumination selon la revendication 6, **caractérisé en ce que** l'unité de commande (8) est configurée pour commander alternativement la première et la seconde partie des pixels LED (4a, ..., 4n) en tant qu'éléments de capteur et en tant qu'éléments d'émission de lumière.

8. Dispositif d'illumination selon l'une quelconque des revendications 5 à 7, **caractérisé en ce que** l'unité de commande (8) est configurée pour commander une pluralité de pixels LED (4a, ..., 4n) répartis sur la surface d'émission (4) de manière essentiellement uniforme et/ou selon un modèle de calibrage prédéfini en tant qu'éléments de capteur.

9. Dispositif d'illumination selon l'une quelconque des revendications 5 à 8, **caractérisé en ce que** l'unité de commande (8) est configurée pour commander au moins chaque quatrième, de préférence chaque troisième pixel LED de la surface d'émission (4) dans une direction de ligne (Z) et/ou une direction de colonne (S) de la surface d'émission (4) du réseau matriciel LED (3) en tant qu'élément de capteur.

10. Dispositif d'illumination selon l'une quelconque des revendications 5 à 9, **caractérisé en ce que** l'unité de commande (8) dans le premier mode de calibrage est configurée pour commander séquentiellement les pixels LED commandés pour l'émission de lumière d'une manière s'élargissant progressivement radialement vers l'extérieur à partir d'un centre de la surface d'émission, notamment essentiellement concentriquement ou en forme d'étoile.

11. Dispositif d'illumination selon l'une quelconque des revendications 1 à 4, **caractérisé en ce que** le moyen de détection comprend une optique, notamment un séparateur de faisceau ou un miroir semi-transparent, disposée entre la surface d'émission (4) et la surface de transmission (2a) du guide de lumière (2), et une unité de capteur associée (11), notamment une caméra CCD ou une matrice de photodiodes, pour détecter le rayonnement réfléchi (R) par des parties (7a, ....7n) non guidant du guide de lumière (2).

12. Dispositif d'illumination selon l'une quelconque des revendications précédentes, **caractérisé en ce que** le réseau matriciel LED (3) comprend de préférence entre 500 et 4500, plus préférentiellement entre 1000 et 4500 pixels LED (4a, ..., 4n), qui sont de préférence formés par des puces LED émettant de la lumière blanche.

13. Dispositif d'illumination selon l'une quelconque des revendications précédentes, **caractérisé en ce que** la connexion (5) de guide de lumière est configurée pour positionner, de préférence au centre, au moins deux guides de lumière (2, 2') ayant des surfaces de transmissions différentes.

14. Procédé de couplage de la lumière dans un guide de lumière pour un endoscope, le procédé comprenant les étapes suivantes :
- l'étape de fournir un dispositif d'illumination (10) comprenant un moyen d'illumination (1) pour coupler la lumière dans un guide de lumière (2) connectable au dispositif, le moyen d'illumination comprenant un réseau matriciel LED (3) comprenant une pluralité de pixels LED (4a, ..., 4n) formant une surface d'émission (4),
- l'étape de positionner une surface de transmission (2a) qui est disposée à une extrémité d'un guide de lumière (2), essentiellement orthogonalement à une direction d'émission principale (L) de la surface d'émission (4) du réseau matriciel LED (3), **caractérisé en ce que** le procédé comprend en outre les étapes suivantes :
- l'étape de détecter les pixels LED de la surface d'émission (4) qui sont dirigés vers des parties (7a, ..., 7n) non guidant la lumière du guide de lumière (2) dans la direction d'émission principale, et
- l'étape de coupler la lumière dans le guide de lumière (2), les pixels LED de la surface d'émission (4) étant sélectivement commandés pour l'émission de lumière, au moins certains des pixels LED dirigés vers les parties (7a, ..., 7n) ne guidant pas la lumière étant commandés à une puissance inférieure ou désactivés.

15. Procédé selon la revendication 14, **caractérisé en ce que** la détection comprend la commande d'une première partie des pixels LED de la surface d'émission pour l'émission de lumière et la commande d'une deuxième partie des pixels LED de la surface d'émission en tant qu'éléments de capteur pour détecter la lumière réfléchie incidente sur le pixel LED respectif.

16. Procédé selon la revendication 15, **caractérisé en ce que** la détection comprend une commande des pixels LED respectifs de manière variable en tant qu'éléments d'émission de lumière (9a, ..., 9n) ou en tant qu'éléments de capteur (9a', ..., 9n'), de préférence selon un ordre chronologique prédéfini et/ou une disposition spatiale des pixels LED respectifs sur la surface d'émission.

17. Procédé selon la revendication 15 ou la revendication 16, **caractérisé en ce que** la commande comprend la commande d'une première partie des pixels LED pour l'émission de lumière et le fonctionnement d'une seconde partie des pixels LED en tant qu'éléments de capteur, essentiellement en forme d'échiquier.

18. Procédé selon l'une quelconque des revendications 15 à 17, **caractérisé en ce que** la commande comprend une commande d'une première partie des pixels LED pour l'émission de lumière d'une manière s'élargissant radialement vers l'extérieur à partir d'un centre de la surface d'émission, en particulier essentiellement concentriquement ou en forme d'étoile.

19. Procédé selon l'une quelconque des revendications 15 à 18, **caractérisé en ce que** la détection comprend une détection d'un contour extérieur (2b) du guide de lumière (2) par l'évaluation des données de capteur acquises par les pixels LED commandés comme éléments de capteur, dans lequel, pendant le couplage de la lumière, les pixels LED sont sélectivement commandés de telle sorte qu'au moins certains des pixels LED dirigés dans la direction d'émission principale (L) vers le contour extérieur (2b) et/ou vers une zone située radialement à l'extérieur du contour extérieur (2b) sont commandés à une puissance inférieure ou désactivés.

20. Utilisation du dispositif d'illumination selon l'une quelconque des revendications 1 à 13 pour coupler la lumière dans au moins deux guides de lumières (2, 2') ayant des surfaces de transmission (2a, 2a') différant par la taille et/ou la forme.
